(19) Europäisches Patentamt
European Patent Office
Office européen des brevets

(11) Numéro de publication : **0 061 982**
**B1**

(12)
# FASCICULE DE BREVET EUROPÉEN

(45) Date de publication du fascicule du brevet : **17.10.84**

(51) Int. Cl.³ : **C 07 D213/06**, C 07 D213/08

(21) Numéro de dépôt : **82420042.2**

(22) Date de dépôt : **30.03.82**

(54) **Procédé de préparation de pyridine et de pyridines substituées.**

(30) Priorité : **01.04.81 FR 8106692**

(43) Date de publication de la demande : **06.10.82 Bulletin 82/40**

(45) Mention de la délivrance du brevet : **17.10.84 Bulletin 84/42**

(84) Etats contractants désignés : **AT BE CH DE FR GB IT LI LU NL SE**

(56) Documents cités :
**EP-A- 0 046 897**
**DE-B- 1 192 648**
**FR-A- 1 102 356**
**FR-A- 2 039 535**
**FR-A- 2 356 638**
**GB-A- 1 011 322**
**GB-A- 1 393 086**

(73) Titulaire : **RHONE-POULENC SPECIALITES CHIMIQUES**
**"Les Miroirs" 18, Avenue d'Alsace**
**F-92400 Courbevoie (FR)**

(72) Inventeur : **Cordier, Georges**
**50, Chemin des Hermières**
**F-69340 Francheville (FR)**
Inventeur : **Leroux, Patrick**
**68, rue de Paris**
**F-92190 Meudon (FR)**

(74) Mandataire : **Vignally, Noel et al**
**RHONE-POULENC RECHERCHES Service Brevets Chimie et Polymères Centre de recherches de Saint-Fons B.P. 62**
**F-69192 St-Fons Cedex (FR)**

**EP 0 061 982 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention concerne un procédé de préparation en phase vapeur de pyridine et de pyridines substituées, à partir de pipéridine, éventuellement substituée, de diamino-1,5 pentane éventuellement substitué, de produits plus lourds tels que des N,N'-bis(pipéridinyle-2) hydrazines comportant éventuellement des substituants sur les cycles pipéridines ou de mélanges de plusieurs de ces composés.

Des procédés d'obtention de pyridine par déshydrogénation catalytique en phase vapeur de pipéridine ont déjà été divulgués dans l'art antérieur.

Ainsi le brevet français n° 1 102 356 décrit un procédé de déshydrogénation catalytique de pipéridine en pyridine par passage de vapeurs de pipéridine et d'hydrogène sur un catalyseur au platine ou au palladium déposé sur un gel de silice, à une température de 200 à 500 °C.

Le gel de silice servant de support n'est pas défini par ses caractéristiques telles que le diamètre des pores ou le volume poreux ; or, des essais montrent que tous les supports ne sont pas équivalents pour l'obtention de bons résultats.

La demande allemande examinée n° 1 192 648 décrit également un procédé de préparation de pyridine par déshydrogénation catalytique de pipéridine en phase vapeur, sur un catalyseur à base de platine ou de palladium déposé sur un gel de silice, à une température située entre 200 400 °C.

La productivité semble faible pour ce procédé d'après les valeurs indiquées dans les exemples.

Enfin le brevet français n° 2 309 535 décrit un procédé d'obtention de méthyl-3 pyridine à partir de méthyl-2 diamino-1,5 pentane et de méthyl-3 pipéridine, à une température comprise entre 200 et 400 °C, en présence de palladium, de platine ou de nickel déposé sur de l'alumine, du kieselguhr, de la ponce ou sur des supports analogues. La productivité en méthyl-3 pyridine par heure et par litre de catalyseur diminue fortement après quelques heures d'opération pour atteindre des valeurs faibles, rendant le procédé peu intéressant économiquement.

Il a maintenant été trouvé, ce qui constitue un objet de la présente invention, un procédé de préparation, avec une excellente productivité, de pyridine et de pyridines substituées, en phase vapeur par cyclisation et/ou déshydrogénation de diamino-1,5 pentane éventuellement substitué, de pipéridine éventuellement substituée ou d'autres composés tels que les N,N'-bis(pipéridinyle-2) hydrazines, en utilisant un catalyseur constitué par du palladium, du platine ou du ruthénium déposés sur un support solide macroporeux.

Plus précisément le procédé selon l'invention est un procédé de préparation de composés de formule générale (I) :

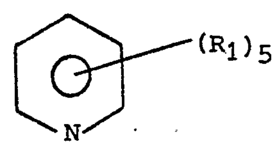

(I)

dans laquelle les radicaux $R_1$, identiques ou différents, représentent un atome d'hydrogène ou un radical alkyle, linéaire ou ramifié ayant 1 à 4 atomes de carbone, par chauffage en phase vapeur :

— d'un composé de formule générale (II)

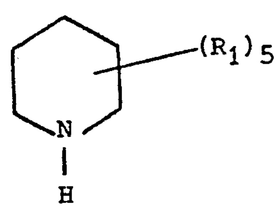

(II)

dans laquelle les radicaux $R_1$ ont les mêmes significations que précédemment,

— ou de diamino-1,5 pentane,

— ou de diamino-1,5 pentane comportant dans sa partie hydrocarbonée de 1 à 3 radicaux alkyles linéaires ou ramifiés ayant de 1 à 4 atomes de carbone,

— ou d'un composé de formule générale (III) :

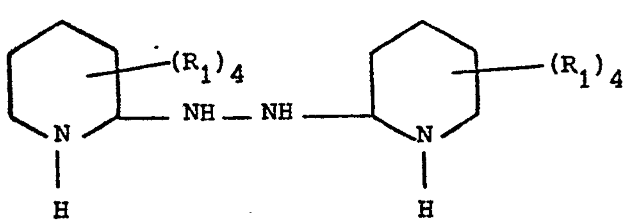

(III)

dans laquelle les radicaux $R_1$ ont les significations indiquées précédemment,

— ou d'un mélange de plusieurs des composés précédents, ledit procédé étant caractérisé en ce que l'on opère en présence d'un catalyseur constitué par du palladium, du platine ou du ruthénium déposé sur un support solide macroporeux dont le diamètre moyen des pores est supérieur à 200 Angstroms et dont le volume poreux est compris entre 0,5 et 1,4 centimètres-cubes/gramme.

De préférence, on utilise un support macroporeux dont le diamètre moyen des pores est compris entre 300 Angstroms et 5 000 Angstroms et dont le volume poreux est compris entre 0,8 et 1,2 $cm^3/g$.

Le support solide macroporeux peut être tout solide utilisé habituellement comme support de catalyseur à la condition que le diamètre moyen de ses pores prenne l'une des valeurs indiquées prédédemment.

C'est ainsi que l'on peut utiliser comme support par exemple l'alumine, le charbon ou la silice.

La silice convient tout particulièrement bien car on peut préparer à volonté des microbilles de silice présentant les caractéristiques souhaitées indiquées ci-avant. Les silices ayant un diamètre moyen de pores compris entre 300 A et 2 000 A et un volume poreux compris entre 0,8 et 1,2 $cm^3/g$, sont préférées.

Pour la préparation de telles silices on peut notamment se référer à la technique décrite dans le brevet français n° 2.093.176 relatives à un traitement en autoclave en milieu à réaction ammoniacale d'hydrogels siliceux séchés.

Le contenu de ce brevet est incorporé à la présente description par référence.

La granulométrie du support utilisé pour la préparation du catalyseur n'est pas un paramètre critique. Elle dépend essentiellement des dimensions du réacteur.

Les techniques de dépôt du métal sur le support macroporeux sont bien connues de l'homme du métier.

Parmi les métaux cités précédemment, on préfère généralement utiliser le palladium déposé sur silice, sur alumine ou sur charbon. Le palladium déposé sur les silices précédemment décrites convient tout particulièrement bien comme catalyseur dans le procédé selon l'invention.

Lorsque l'on veut préparer un catalyseur Pd/silice on peut effectuer par exemple une imprégnation par échange en utilisant des solutions ammoniacales de chlorure de palladium tétramine en présence de chlorure d'ammonium.

Le rapport pondéral métal/support solide macroporeux est en général inférieur ou égal à 10 %. Ce rapport est le plus souvent compris entre 0,1 % et 5 %. Il est de préférence situé entre 0,2 % et 2 % pour les catalyseurs Pd/silice.

En règle générale lorsqu'on utilise des supports dont le diamètre moyen des pores tend vers les valeurs les plus faibles indiquées, il est favorable d'utiliser des rapports pondéraux métal/support situés dans la partie inférieure des zones indiquées précédemment.

La quantité de catalyseur rapportée au composé à cycliser et/ou à déshydrogéner (ou substrat) mis en œuvre n'est pas critique. Toutefois comme l'on utilise le plus souvent un lit de catalyseur dans lequel le substrat circule à l'état de vapeur, la quantité de la couche de catalyseur doit être suffisante pour assurer le taux de transformation souhaité compte tenu du temps de contact entre les vapeurs de substrat et le catalyseur.

La température à laquelle est mis en œuvre le procédé selon l'invention doit être suffisante pour que les réactifs de départ soient à l'état de vapeurs et pour que la réaction soit suffisamment rapide. On a constaté que des températures de 200° à 500 °C conviennent bien. De préférence on opère à des températures comprises entre 250 °C et 400 °C.

Lors de la mise en œuvre du procédé selon l'invention il est généralement favorable d'injecter de l'hydrogène dans le réacteur où se produira la réaction de cyclisation et/ou de déhydrogénation, simultanément à l'injection du substrat à traiter. Le rapport molaire hydrogène/substrat n'est pas critique et peut varier dans de très larges limites. Il peut être compris par exemple entre 0,1 et 500. Mais il n'est guère utile d'adopter un rapport trop élevé qui réduit la pression partielle du substrat et diminue son temps de séjour dans le réacteur.

Habituellement des rapports molaires hydrogène/substrat de 0,5 à 10 conviennent bien.

Les composés auxquels le procédé selon l'invention peut être notamment appliqué sont :

— la pipéridine ;

— la pipéridine dont le cycle est substitué par un ou plusieurs radicaux méthyle ou éthyle ;

— le diamino-1,5 pentane ;

— le diamino-1,5 pentane dont la partie hydrocarbonée comporte un ou plusieurs substituants méthyle ou éthyle ;

— la N,N'bis(pipéridinyle-2) hydrazine ;

— la N,N'bis(pipéridinyle-2) hydrazine dont un cycle pipéridinyle ou les deux cycles pipéridinyles sont porteurs d'un ou plusieurs radicaux méthyle ou éthyle ;

— des mélanges de plusieurs des composés précédents.

Parmi ces composés, le procédé selon l'invention convient tout particulièrement bien à :

— la pipéridine ;

— la pipéridine dont le cycle est substitué par un ou deux radicaux méthyle ou éthyle comme par exemple :

la méthyl-3 pipéridine, la méthyl-2 pipéridine,

la méthyl-4 pipéridine, l'éthyl-2 pipéridine,
l'éthyl-3 pipéridine, l'éthyl-4 pipéridine,
la diméthyl-2,3 pipéridine, la diméthyl-2,4 pipéridine,
la diméthyl-2,5 pipéridine, la diméthyl-3,4 pipéridine,
la diméthyl-3,5 pipéridine, la diéthyl-2,3 pipéridine,
la diéthyl-2,4 pipéridine, la diéthyl-2,5 pipéridine,
la diéthyl-3,4 pipéridine, la diéthyl-3,5 pipéridine,
l'éthyl-2 méthyl-3 pipéridine, l'éthyl-3 méthyl-2 pipéridine,
l'éthyl-2 méthyl-4 pipéridine, l'éthyl-4 méthyl-2 pipéridine,
l'éthyl-2 méthyl-5 pipéridine, l'éthyl-5 méthyl-2 pipéridine,
l'éthyl-3 méthyl-4 pipéridine, l'éthyl-4 méthyl-3 pipéridine,
l'éthyl-3 méthyl-5 pipéridine, l'éthyl-5 méthyl-3 pipéridine ;

— le diamino-1,5 pentane ;

— les diamino-1,5 pentanes dont la partie hydrocarbonée comporte un ou 2 substituants méthyle ou éthyle comme par exemple :

le méthyl-1 diamino-1,5 pentane,
le méthyl-2 diamino-1,5 pentane,
le méthyl-3 diamino-1,5 pentane,
l'éthyl-1 diamino-1,5 pentane,
l'éthyl-2 diamino-1,5 pentane,
l'éthyl-3 diamino-1,5 pentane,
le diméthyl-1,2 diamino-1,5 pentane,
le diméthyl-1,3 diamino-1,5 pentane,
le diméthyl-1,4 diamino-1,5 pentane,
le diméthyl-2,3 diamino-1,5 pentane,
le diméthyl-2,4 diamino-1,5 pentane ;

— la N,N'bis(pipéridinyle-2) hydrazine ;

— la N,N'bis(pipéridinyle-2) hydrazine dont un cycle pipéridinyle ou les deux cycles pipéridinyles sont porteurs d'un ou deux radicaux méthyle ou éthyle comme par exemple :

la N,N'bis(méthyl-3 pipéridinyle-2) hydrazine,
la N(méthyl-3 pipéridinyle-2) N'(méthyl-5 pipéridinyle-2) hydrazine,
la N,N'bis(méthyl-5 pipéridinyle-2) hydrazine,
la N,N'bis(éthyl-3 pipéridinyle-2) hydrazine,
la N,N'bis(éthyl-5 pipéridinyle-2) hydrazine.

On peut bien entendu utiliser des mélanges de plusieurs des composés précédents, notamment les mélanges qui conduisent par cyclisation et/ou déshydrogénation au même composé de formule (I) final.

Le catalyseur utilisé dans le procédé selon l'invention est très intéressant, car il permet d'obtenir la pyridine ou les pyridines substitutées par des radicaux méthyle et/ou éthyle à partir de la pipéridine, du diamino-1,5 pentane ou de leurs dérivés méthylés et/ou éthylés, ou à partir de composés dits lourds provenant de la condensation de composés intermédiaires obtenus lors de l'hydrogénation du glutaronitrile ou du glutaronitrile substitué par des radicaux méthyle et/ou éthyle.

C'est ainsi que l'on peut en particulier appliquer le procédé selon l'invention au mélange de composés obtenus lors de l'hydrogénation du méthyl-2 glutaronitrile, sans qu'il soit nécessaire d'isoler ses différents constituants. Cela permet en outre de valoriser les produits formés par condensation de composés intermédiaires.

Ainsi lorsque l'on hydrogène le méthyl-2 glutaronitrile sur du nickel de Raney, on obtient essentiellement un mélange de méthyl-2 diamino-1,5 pentane, de méthyl-3 pipéridine, de la N,N'bis(méthyl-3 pipéridinyle-2) hydrazine, de la N,N'bis(méthyl-5 pipéridinyle-2) hydrazine, et de la N-(méthyl-3 pipéridinyle-2) N'-(méthyl-5 pipéridinyle-2) hydrazine que l'on peut traiter directement par le procédé selon l'invention.

Le procédé selon l'invention s'applique donc de préférence à ces produits d'hydrogénation du méthyl-2 glutaronitrile, aux produits d'hydrogénation du glutaronitrile comme le diamino-1,5 pentane, la pipéridine et la N,N'bis(pipéridinyle-2) hydrazine, à l'éthyl-5 méthyl-2 pipéridine et à des mélanges de plusieurs de ces composés, ce qui conduit finalement soit à la β-picoline, soit à la pyridine, soit à l'éthyl-5 méthyl-2 pyridine, soit à des mélanges de la pyridine et de ses dérivés.

L'appareillage utilisé pour la mise en œuvre du procédé selon l'invention n'est pas spécifique. On peut utiliser tout appareillage permettant d'opérer en phase vapeur.

D'un point de vue pratique on peut par exemple opérer de la manière suivante pour la mise en œuvre du procédé selon l'invention. Dans un réacteur approprié on dépose le catalyseur en couche d'épaisseur déterminée en fonction du débit de substrat à traiter. Cette couche de catalyseur peut être également disposée soit sous une couche d'un solide inerte, comme le quartz, ayant une granulométrie du même ordre de grandeur que celle du catalyseur, soit entre deux couches d'un tel solide inerte ; la couche de catalyseur peut également être mélangée à un tel solide inerte.

La couche de catalyseur et les éventuelles couches de solide inerte sont portées à la température désirée.

Le substrat à traiter et l'hydrogène sont injectés simultanément dans une enceinte portée à une température suffisante pour vaporiser ledit substrat.

Les produits de la réaction sont ensuite condensés dans une enceinte à basse température comportant éventuellement un solvant de ces produits.

Le traitement de la masse réactionnelle finale est effectuée selon les méthodes classiques de la chimie et les dosages de ses différents composés sont faits par exemple par chromatographie en phase vapeur.

Le catalyseur du procédé selon l'invention conserve une bonne activité même après plusieurs centaines d'heures d'utilisation. On peut cependant, lorsqu'on le juge nécessaire, le réactiver, par exemple en le portant à une température supérieure à celle à laquelle il était utilisé.

Les produits obtenus par le procédé selon l'invention ont des usages divers. La pyridine sert notamment le solvant. La β-picoline (ou méthyl-3 pyridine) sert à la préparation de l'acide nicotinique ou du nicotinamide utilisés en pharmacie et en alimentation. L'éthyl-5 méthyl-2 pyridine sert également d'intermédiaire pour la préparation de l'acide nicotinique. Les exemples qui suivent sont donnés à titre d'illustration de l'invention. Afin de mieux définir les performances des catalyseurs utilisés dans le procédé selon l'invention et notamment afin de comparer leur activité et leur longévité à celles des catalyseurs ne présentant pas leurs caractéristiques, notamment les catalyseurs utilisés dans les procédés de l'art antérieur, nous avons déterminé dans les exemples suivants, outre le taux de transformation des substrats utilisés et le rendement obtenu en composé de formule (I), la productivité du catalyseur. Cette productivité est exprimée en grammes de composé de formule (I) obtenu par heure et par litre de catalyseur mis en œuvre.

Exemple 1

Dans un microréacteur en verre ayant une hauteur de 200 mm et un diamètre de 15 mm et placé verticalement, on dispose successivement de bas en haut :

— une première couche de 65 mm d'épaisseur de quartz inerte ayant une granulométrie comprise entre 250 microns et 315 microns (volume de la couche 10 $cm^3$) ;

— une couche de catalyseur consistant en 0,6 % en poids de palladium déposé sur des microbilles de silice (granulométrie de 150 à 300 microns) ayant un volume poreux de 0,94 $cm^3$ par gramme présentant un diamètre moyen de pores d'environ 500 Angstroms et ayant une surface spécifique de 67 $m^2$/gramme (volume de la couche 1 $cm^3$) ;

— une deuxième couche de quartz identique à la première.

Le microréacteur est muni d'une gaine thermométrique centrale, d'une batterie de thermocouples à différents niveaux du lit catalytique afin d'en contrôler la température, d'un système de chauffage électrique et d'un système d'injection par micropompe seringue à son extrémité supérieure.

A la partie inférieure du microréacteur se trouve un récepteur contenant de l'éthanol maintenu à − 15 °C, dans lequel seront condensés les produits de la réaction.

La température de la couche catalytique est régulée à 270 °C. Par le haut du réacteur on injecte conjointement de l'hydrogène avec un débit de 1,3 litre/heure (volume calculé pour les conditions de température et de pression normales : TPN) et de la méthyl-3 pipéridine avec un débit d'environ exactement 2,0 grammes/heure, dans la chambre d'injection constituant la partie supérieure du réacteur et qui est à 150 °C. La méthyl-3 pipéridine est instantanément vaporisée dans cette chambre d'injection. Les produits de la réaction sont condensés et recueillis dans l'éthanol et la solution obtenue est analysée par chromatographie en phase vapeur.

On calcule le taux de transformation (TT) de la méthyl-3 pipéridine, le rendement (RT) en β-picoline (métyl-3 pyridine) obtenue par rapport à la méthyl-3 pipéridine transformée et la productivité en grammes de β-picoline par heure et par litre de catalyseur.

Le tableau 1 ci-après rassemble les valeurs instantanées du TT et du RT ainsi que la productivité calculée pour différentes durées d'opération.

Tableau 1

| Durée en heures | TT % de la méthyl-3 pipéridine | RT % en β-picoline | Productivité en β-picoline (en g/h / litre) |
|---|---|---|---|
| 1 | 98 | 100 | 2000 |
| 20 | 75 | 100 | 1400 |
| 80 | 48 | 100 | 920 |
| 240 | 30 | 100 | 550 |
| 320 | 30 | 100 | 550 |

Ensuite on porte la température de la couche catalytique à environ 330 °C. (Chambre d'injection : 210 °C).

On maintient le débit d'hydrogène à sa valeur précédente et on passe le débit de la méthyl-3 pipéridine à environ exactement 5,5 g/heure. Le tableau 2 ci-après rassemble les valeurs du TT, du RT et de la productivité pour différentes durées de réaction à environ 330 °C (entre parenthèses on indique la durée totale de l'essai, c'est-à-dire les 320 heures de fonctionnement à 270 °C augmentées des heures de fonctionnement à environ 330 °C).

Tableau 2

| Durée en heures | TT % | RT % en ß-picoline | Productivité en ß-picoline (g/h / litre) |
|---|---|---|---|
| 1 (321) | 60 | 100 | 3300 |
| 40 (360) | 65 | 100 | 3580 |
| 100 | 67 | 100 | 3630 |

Le taux de transformation est maintenu volontairement inférieur à 100 % afin de connaître la productivité maximale à un instant donné et pouvoir ainsi enregistrer les éventuelles baisses de cette productivité.

Exemple 2

On utilise le même appareillage et le même mode opératoire que dans l'exemple 1.

La couche catalytique est constituée comme dans l'exemple 1 et avec le même lot de catalyseur, mais elle est maintenue à 300 °C (chambre d'injection : 180 °C).

Le débit d'hydrogène est de 3,7 litres/heure (TPN) et le débit de la méthyl-3 pipéridine injectée est de 5,0 g/heure. La durée de l'essai est de 1 heure. On obtient les résultats suivants :

— TT de la méthyl-3 pipéridine = 98 %
— RT en β-picoline = 99 %
— Productivité en β-picoline = 4 560 g/heure par litre de catalyseur.

Exemple 3

On utilise le même appareillage et le même mode opératoire que dans l'exemple 1.

La couche catalytique est constituée comme dans l'exemple 1 et avec le même lot de catalyseur, mais elle est maintenue à 350 °C. (Chambre d'injection : 230 °C).

Le débit d'hydrogène est de 3,7 litres/heure (TPN) et le débit de la méthyl-3 pipéridine injectée est de 12,0 g/heure. La durée de l'essai est de 1 heure. On obtient les résultats suivants :

— TT de la méthyl-3 pipéridine = 96 %
— RT en β-picoline = 99 %
— Productivité en β-picoline = 10 700 g/heure par litre de catalyseur.

Exemple 4

On utilise le même appareillage et le même mode opératoire que dans l'exemple 1.

La couche catalytique est constituée comme dans l'exemple 1 et avec le même lot de catalyseur, mais elle est maintenue à 300 °C. (Chambre d'injection : 180 °C).

Le débit d'hydrogène est de 3,7 litres/heure (TPN) et on injecte conjointement du méthyl-2 diamino-1,5 pentane avec un débit de 1,4 g/h. La durée de l'essai est de 1 heure. On obtient les résultats suivants :

— TT de la méthyl-2 diamino-1,5 pentane = 100 %
— RT en β-picoline = 75 %
— RT en méthyl-3 pipéridine = 25 %.

Exemple 5

On utilise le même appareillage et le même mode opératoire que dans l'exemple 1.

La couche catalytique est constituée comme dans l'exemple 1 et avec le même lot de catalyseur, mais elle est maintenue à 350 °C. (Chambre d'injection : 230 °C).

Le débit d'hydrogène est de 3,7 litres/heure (TPN) et on injecte conjointement des composés « lourds » issus de l'hydrogénation du méthyl-2 glutaronitrile et ayant la formule générale :

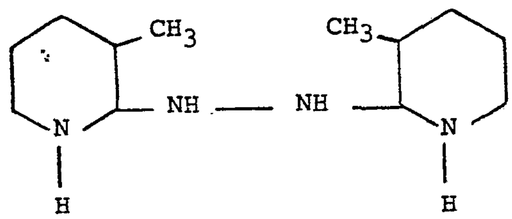

avec un débit de 4,0 g/h. La durée de l'essai est de 1 heure.

On obtient les résultats suivants :

— TT desdits composés « lourds » = 100 %
— RT en β-picoline = 85 %
— RT en méthyl-3 pipéridine = 3 %.

Exemple 6

On répète l'exemple 5 en injectant avec un débit d'environ exactement 1,3 g/h un mélange brut issu de l'hydrogénation de méthyl-2 glutaronitrile, en présence d'ammoniac, sur du nickel de Raney, sous une pression totale ($NH_3$ + Hydrogène) de 50 bars, puis de 75 bars et à une température de 80 °C, puis de 100 °C ; ce mélange a la composition suivante :

méthyl-3 pipéridine = 59 % en poids
méthyl-2 diamino-1,5 pentane = 31,7 % en poids
composés « lourds » tels que ceux traités dans l'exemple 5 = 9,3 % en poids.

Le tableau 3 rassemble les compositions de la masse réactionnelle finale (en % en poids) après différentes durées de fonctionnement et la productivité obtenue en β-picoline.

Tableau 3

| Durée en heures | % ß-picoline | % méthyl-3 pipéridine | % méthyl-2 diamino-1,5 pentane | % "lourds" | Productivité en ß-picoline g/h / litre de catalyseur |
|---|---|---|---|---|---|
| 95 | 82,7 | 10,5 | 3,9 | 2,9 | 760 |
| 261 | 44,7 | 41,3 | 10,8 | 3,2 | 480 |
| 457 | 65,3 | 23,8 | 8,0 | 2,9 | 625 |

Essais comparatifs

Ces essais sont effectués dans les mêmes conditions que les exemples précédents, mais en utilisant, soit des catalyseurs du commerce, soit un catalyseur dont le support ne répond pas aux caractéristiques de macroporosité des catalyseurs du procédé selon l'invention.

Essai A

On utilise le même appareillage et le même mode opératoire que dans l'exemple 1.

La couche catalytique est constituée comme dans l'exemple 1 mais en utilisant comme catalyseur du palladium déposé à 0,5 % sur une alumine ayant une granulométrie de 250 à 315 microns, un volume poreux de 0,30 $cm^3$/gramme, un diamètre moyen des pores de 28 Angstroms et une surface spécifique de 220 $m^2$ par gramme.

La couche catalytique est maintenue à 300 °C. (Chambre d'injection : 180 °C).

Le débit d'hydrogène est de 3,7 l/h (TPN) et on injecte conjointement de la méthyl-3 pipéridine avec un débit de 0,33 g/h.

Le tableau 4 rassemble les valeurs du TT, du RT et de la productivité pour différentes durées de fonctionnement.

Tableau 4

| Durée en heures | TT % de la méthyl-3 pipéridine | RT % en ß-picoline | Productivité en ß-picoline (g/h / litre) |
|---|---|---|---|
| 1 | 95 | 100 | 295 |
| 3 | 55 | 100 | 170 |
| 20 | 25 | 100 | 77 |
| 30 | 10 | 100 | 30 |

On note des valeurs de productivité plus faibles qu'avec le catalyseur utilisé dans les exemples 1 à 6 et une désactivation rapide de ce catalyseur se traduisant par une diminution corrélative de ladite productivité.

Essai B

On reproduit l'essai A, mais en remplaçant le $Pd/Al_2O_3$ par un catalyseur vendu par la Société ENGELHARD qui est du Pd déposé à 0,5 % enpoids sur de la silice ayant un volume poreux de 1,04 $cm^3/g$, un diamètre moyen des pores de 100 Angstroms et une surface spécifique de 293 $m^2$ par gramme.

La température de la couche catalytique est de 300 °C. (Chambre d'injection : 180 °C).

Le débit d'hydrogène est de 3,7 l/h (TPN) et on injecte conjointement 1,7 g/h de méthyl-3 pipéridine.

Le tableau 5 rassemble les valeurs du TT, du RT et de la productivité pour différentes durées de fonctionnement.

Tableau 5

| Durée en heures | TT % de la méthyl-3 pipéridine | RT % en ß-picoline | Productivité en ß-picoline (g/h / litre) |
|---|---|---|---|
| 7 | 14,1 | 100 | 105 |
| 23 | 11 | 100 | 81 |
| 46 | 7,4 | 100 | 94 |
| 63 | 3,2 | 100 | 63 |

Dans cet essai on note encore une faible productivité initiale qui décroît très rapidement.

Essai C

On utilise le même appareillage et le même mode opératoire que dans l'exemple 1.

La couche catalytique est constituée comme dans l'exemple 1 mais en utilisant comme catalyseur du palladium déposé à 1,6 % sur une silice ayant une granulométrie de 150 à 300 microns, un volume poreux de 0,91 $cm^3/g$, un diamètre moyen de pores de 80 A et une surface spécifique de 375 $m^2/g$.

La couche catalytique est maintenue à 270 °C. (Chambre d'injection : 150 °C).

Le débit d'hydrogène est de 3,7 l/h (TPN) et on injecte conjointement de la méthyl-3 pipéridine avec un débit d'environ exactement 5,32 g/h pendant une heure ; puis on poursuit l'injection conjointe avec un débit d'hydrogène de 1 litre/heure (TPN) et un débit de méthyl-3 pipéridine d'environ exactement 1,4 g/h.

Le tableau 6 rassemble les valeurs du TT, du RT et de la productivité pour deux durées de fonctionnement.

Tableau 6

| Durée en heures | TT % de la méthyl-3 pipéridine | RT % en ß-picoline | Productivité en ß-picoline (g/h / litre) |
|---|---|---|---|
| 1 | 67 | 100 | 3350 |
| 3,5 | 75 | 100 | 1000 |

On note une décroissance très rapide de la productivité.

**Revendications**

1. Procédé de préparation de composés de formule générale (I) :

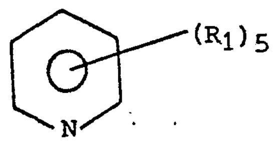

(I)

dans laquelle les radicaux $R_1$, identiques ou différents, représentent un atome d'hydrogène ou un radical alkyle, linéaire ou ramifié ayant 1 à 4 atomes de carbone, par chauffage en phase vapeur à une température de 200 °C à 500 °C :

— d'un composé de formule générale (II) :

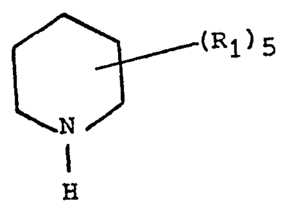

(II)

dans laquelle les radicaux $R_1$ ont les mêmes significations que précédemment,

— ou de diamino-1,5 pentane,

— ou de diamino-1,5 pentane comportant dans sa partie hydrocarbonée de 1 à 3 radicaux alkyles linéaires ou ramifiés ayant de 1 à 4 atomes de carbone,

— ou d'un composé de formule générale (III) :

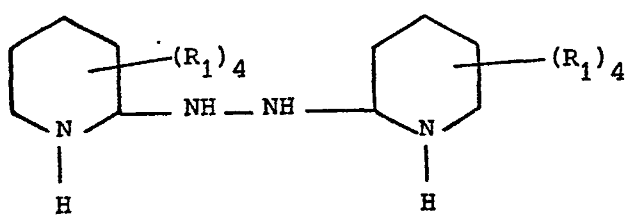

(III)

dans laquelle les radicaux $R_1$ ont les significations indiquées précédemment,

— ou d'un mélange de plusieurs des composés précédents, ledit procédé étant caractérisé en ce que l'on opère en présence d'un catalyseur constitué par du palladium, du platine ou du ruthénium déposé sur un support solide macroporeux dont le diamètre moyen des pores est supérieur à 200 Angstroms et dont le volume poreux est compris entre 0,5 et 1,4 centimètres-cubes/gramme.

2. Procédé selon la revendication 1, caractérisé en ce que le support solide macroporeux est une silice, une alumine ou du charbon dont le diamètre moyen des pores est compris entre 300 A et 5 000 A et dont le volume poreux est compris entre 0,8 et 1,2 $cm^3$/gramme.

3. Procédé selon l'une des revendications 1 et 2, caractérisé en ce que le métal utilisé dans le catalyseur est le palladium.

4. Procédé selon l'une quelconque des revendications 1 à 3, caractérisé en ce que le catalyseur utilisé est du palladium déposé sur une silice dont le diamètre moyen des pores est compris entre 300 A et 2 000 A et dont le volume poreux est compris entre 0,8 et 1,2 $cm^3$/g.

5. Procédé selon l'une quelconque des revendications 1 à 4, caractérisé en ce que dans le catalyseur le rapport pondéral métal/support solide macroporeux est inférieur ou égal à 10 %.

6. Procédé selon la revendication 4, caractérisé en ce que le rapport pondéral palladium/silice est compris entre 0,2 et 2 %.

7. Procédé selon l'une quelconque des revendications 1 à 6, caractérisé en ce qu'on l'applique à :
— la pipéridine ;
— la pipéridine dont le cycle est substitué par un ou plusieurs radicaux méthyle ou éthyle ;
— le diamino-1,5 pentane ;
— le diamino-1,5 pentane dont la partie hydrocarbonée comporte un ou plusieurs substituants méthyle ou éthyle ;
— la N,N'bis(pipéridinyle-2) hydrazine ;
— la N,N'bis(pipéridinyle-2) hydrazine dont un cycle pipéridinyle ou les deux cycles pipéridinyles sont porteurs d'un ou plusieurs radicaux méthyle ou éthyle ;
— des mélanges de plusieurs des composés précédents.

8. Procédé selon l'une quelconque des revendications 1 à 6, caractérisé en ce qu'on l'applique à :
— la pipéridine ;
— la méthyl-3 pipéridine ;
— l'éthyl-5 méthyl-2 pipéridine ;
— le diamino-1,5 pentane ;
— le méthyl-2 diamino-1,5 pentane ;
— la N,N'bis(pipéridinyle-2) hydrazine ;
— la N,N'bis(méthyl-3 pipéridinyle-2) hydrazine ;
— la N,N'bis (méthyl-5 pipéridinyle-2) hydrazine ;
— la N-(méthyl-3 pipéridinyle-2) N'-(méthyl-5 pipéridinyle-2) hydrazine ;
— ou à des mélanges de plusieurs des composés précédents.

9. Procédé selon l'une quelconque des revendications 1 à 6, caractérisé en ce qu'on l'applique au mélange brut obtenu lors de l'hydrogénation du méthyl-2 glutaronitrile.

10. Procédé selon l'une quelconque des revendications 1 à 9, caractérisé en ce que l'on opère entre 250 °C et 400 °C.

11. Procédé selon l'une quelconque des revendications 1 à 10, caractérisé en ce que l'on injecte simultanément le ou les substrats à traiter et de l'hydrogène.

**Claims**

1. Process for the preparation of compounds of the general formula (I) :

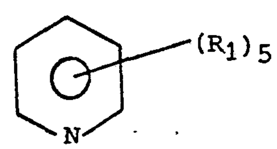

(I)

in which the radicals $R_1$, which are identical or different, represent a hydrogen atom or a linear or branched alkyl radical having 1 to 4 carbon atoms, by heating, in the vapour phase, at a temperature of 200 °C to 500 °C :
— a compound of the general formula (II) :

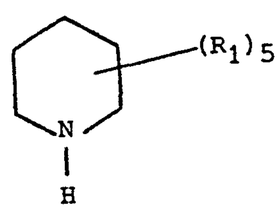

(II)

in which the radicals $R_1$ have the same meanings as above, or
— 1,5-diaminopentane, or
— 1,5-diaminopentane containing, in its hydrocarbon part, from 1 to 3 linear or branched alkyl radicals having from 1 to 4 carbon atoms, or
— a compound of the general formula (III) :

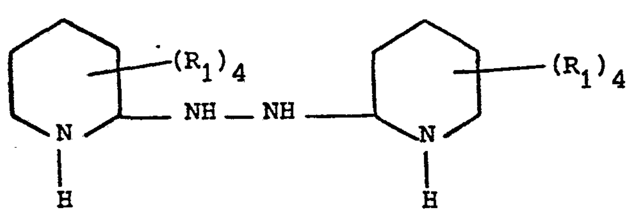

(III)

in which the radicals $R_1$ have the meanings indicated above, or

— a mixture of several of the above compounds, the said process being characterised in that the reaction is carried out in the presence of a catalyst consisting of palladium, platinum or ruthenium deposited on a macroporous solid support of which the average pore diameter is more than 200 Angstroms and of which the porous volume is between 0.5 and 1.4 cubic centimetres/gram.

2. Process according to Claim 1, characterised in that the macroporous solid support is a silica, an alumina or carbon of which the average pore diameter is between 300 A and 5,000 A and of which the porous volume is between 0.8 and 1.2 $cm^3$/gram.

3. Process according to one of Claims 1 and 2, characterised in that the metal used in the catalyst is palladium.

4. Process according to any one of Claims 1 to 3, characterised in that the catalyst used is palladium deposited on a silica of which the average pore diameter is between 300 A and 2,000 A and of which the porous volume is between 0.8 and 1.2 $cm^3$/g.

5. Process according to any one of Claims 1 to 4, characterised in that, in the catalyst, the weight ratio metal/macroporous solid support is less than or equal to 10 %.

6. Process according to Claim 4, characterised in that the weight ratio palladium/silica is between 0.2 and 2 %.

7. Process according to any one of Claims 1 to 6, characterised in that it is applied to :

— piperidine ;

— piperidine of which the ring is substituted by one or more methyl or ethyl radicals ;

— 1,5-diaminopentane ;

— 1,5-diaminopentane of which the hydrocarbon part contains one or more methyl or ethyl substituents ;

— N,N′-bis-(piperidin-2-yl)-hydrazine ;

— N,N′-bis-(piperidin-2-yl)-hydrazine of which one piperidinyl ring or both the piperidinyl rings carry one or more methyl or ethyl radicals ; and

— mixtures of several of the above compounds.

8. Process according to any one of Claims 1 to 6, characterised in that it is applied to : piperidine, 3-methylpiperidine, 5-ethyl-2-methylpiperidine, 1,5-diaminopentane, 2-methyl-1,5-diaminopentane, N,N′-bis-(piperidin-2-yl)-hydrazine, N,N′-bis-(3-methylpiperidin-2-yl)-hydrazine, N,N′-bis-(5-methylpiperidin-2-yl)-hydrazine or N-(3-methylpiperidin-2-yl)-N′-(5-methyl-piperidin-2-yl)-hydrazine, or to mixtures of several of the above compounds.

9. Process according to any one of Claims 1 to 6, characterised in that it is applied to the crude mixture obtained during the hydrogenation of 2-methylglutaronitrile.

10. Process according to any one of Claims 1 to 9, characterised in that the reaction is carrried out at between 250 °C and 400 °C.

11. Process according to any one of Claims 1 to 10, Characterised in that the substrate or substrates to be treated and hydrogen are injected simultaneously.

**Ansprüche**

1. Verfahren zur Herstellung von Verbindungen der allgemeinen Formel (I) :

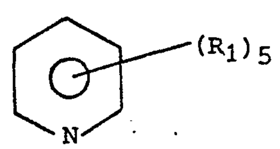

(I)

in welcher die Reste $R_1$ unabhängig voneinander ein Wasserstoffatom oder einen geradkettigen oder verzweigten Alkylrest mit 1 bis 4 Kohlenstoffatomen darstellen, durch Erhitzen

— einer Verbindung der allgemeinen Formel (II) :

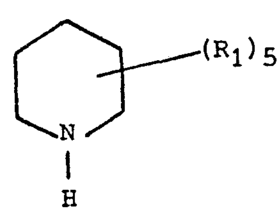

(II)

in welcher die Reste $R_1$ die zuvor angegebenen Bedeutungen haben,

— oder von 1,5-Diaminopentan,

— oder von 1,5-Diamino-pentan, das in seinem Kohlenwasserstoffteil 1 bis 3 geradkettige oder verzweigte Alkylreste mit 1 bis 4 Kohlenstoffatomen enthält,

— oder einer Verbindung der allgemeinen Formel (III) :

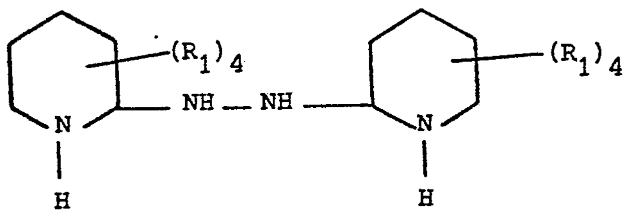

(III)

in welcher die Reste $R_1$ die oben angegebenen Bedeutungen haben,

— oder einer Mischung von mehreren der obigen Verbindungen in Dampfphase bei einer Temperatur von 200 °C bis 500 °C, welches Verfahren dadurch gekennzeichnet ist, daß man in Anwesenheit eines Katalysators arbeitet, der aus Palladium, Platin oder Ruthenium auf einem makroporösen festen Träger besteht, dessen mittlerer Porendurchmesser größer als 200 Ångström (20 nm) ist und dessen Porenvolumen 0,5 bis 1,4 $cm^3/g$ beträgt.

2. Verfahren nach Anspruch 1, dadurch gekennzeichnet, daß der makroporöse feste Träger ein Siliciumdioxid, Aluminiumoxid oder aus Kohle ist, deren mittlerer Porendurchmesser 300 bis 5 000 Å (30 bis 500 nm) beträgt und deren Porenvolumen zwischen 0,8 und 1,2 $cm^3/g$ liegt.

3. Verfahren nach einem der Ansprüche 1 und 2, dadurch gekennzeichnet, daß das im Katalysator verwendete Metall Palladium ist.

4. Verfahren nach einem der Ansprüche 1 bis 3, dadurch gekennzeichnet, daß der verwendete Katalysator aus Palladium besteht, das auf einem Siliciumdioxid abgelagert ist, dessen mittlerer Porendurchmesser 300 bis 2 000 Å (30 bis 200 nm) beträgt und dessen Porenvolumen 0,8 bis 1,2 $cm^3/g$ beträgt.

5. Verfahren nach einem der Ansprüche 1 bis 4, dadurch gekennzeichnet, daß das Gewichtsverhältnis von Metall/makroporösem, festem Träger kleiner oder gleich 10 % ist.

6. Verfahren nach Anspruch 4, dadurch gekennzeichnet, daß das Gewichtsverhältnis Palladium/Siliciumdioxid 0,2 bis 2 % beträgt.

7. Verfahren nach einem der Ansprüche 1 bis 6, dadurch gekennzeichnet, daß man es auf :

— Piperidin ;

— Piperidin, dessen Ring durch eine oder mehrere Methyl- oder Äthylgruppen substituiert ist ;

— 1,5-Diamino-pentan ;

— 1,5-Diamino-pentan, dessen Kohlenwasserstoffteil einen oder mehrere Methyl- oder Äthylsubstituenten trägt ;

— N,N'-bis(2-Piperidinyl)-hydrazin ;

— N,N'-bis(2-Piperidinyl)-hydrazin, dessen einer Piperidinylring oder dessen beide Piperidinylringe einen oder mehrere Methyl- oder Äthylreste tragen ;

— Mischungen mehrerer der obigen Verbindungen anwendet.

8. Verfahren nach einem der Ansprüche 1 bis 6, dadurch gekennzeichnet, daß man es auf :

— Piperidin ;

— 3-Methyl-piperidin ;

— 5-Äthyl-2-methyl-piperidin ;

— 1,5-Diamino-pentan ;

— 2-Methyl-1,5-diamino-pentan ;

— N,N'-bis(2-Piperidinyl)-hydrazin ;

— N,N'-bis(3-methyl-2-piperidinyl)-hydrazin ;

— N,N'-bis(5-methyl-2-piperidinyl)-hydrazin ;

— N-(3-Methyl-2-piperidinyl)-N'-(5-methyl-2-piperidinyl)-hydrazin ;

— oder Mischungen mehrerer der obigen Verbindungen anwendet.

9. Verfahren nach einem der Ansprüche 1 bis 6, dadurch gekennzeichnet, daß man es auf die rohe Mischung anwendet, die man bei der Hydrierung von 2-Methyl-glutaronitril erhält.

10. Verfahren nach einem der Ansprüche 1 bis 9, dadurch gekennzeichnet, daß man zwischen 250 °C und 400 °C arbeitet.

11. Verfahren nach einem der Ansprüche 1 bis 10, dadurch gekennzeichnet, daß man gleichzeitig das oder die zu behandelnden Substrate und Wasserstoff einbläst.